# NOUVEAU FASCICULE DE BREVET EUROPEEN

(11) **EP 1 762 222 B2**
(45) Date de publication et mention de la décision concernant l'opposition: **21.08.2013**
(45) Mention de la délivrance du brevet: 07.10.2009
(21) Numéro de dépôt: 06118382.8
(22) Date de dépôt: 03.08.2006
(51) Int. Cl.: A61K 8/49, A61Q 5/10

(54) **Procédé de coloration des fibres kératiniques comprenant une étape de traitement du cuir chevelu a partir d'un ester de sorbitan particulier**
Verfahren zum Färben von Keratinfasern, das einen Schritt zur Behandlung der Kopfhaut mittels eines besonderen Sorbitanesters umfasst.
Process for colouring keratin fibres comprising a step of treating the scalp with a particular sorbitan ester

(30) Priorité: 11.08.2005 FR 0552494
(43) Date de publication de la demande: 14.03.2007
(73) Titulaire: L'Oréal, 75008 Paris (FR)
(72) Inventeur: Audousset, Marie-Pascale, 92600 Asnière (FR); Rondeau, Christine, 78500 Sartrouville (FR)
(74) Mandataire: Fevrier, Murielle Françoise E.

(56) Documents cités:
- EP-A1- 1 027 878
- EP-A1- 1 132 079
- EP-A2- 0 155 737
- EP-A2- 1 438 947
- WO-A-2004/093835
- WO-A1-88/00185
- DE-A1- 2 356 475
- DE-A1- 10 028 723
- DE-A1- 19 923 438
- US-A1- 2004 151 746
- US-A1- 2005 136 085
- PATENT ABSTRACTS OF JAPAN vol. 015, no. 388 (C-0872), 2 octobre 1991 (1991-10-02) & JP 03 157320 A (YAMAHATSU SANGYO KK), 5 juillet 1991 (1991-07-05)

## Description

La présente invention a pour objet un procédé de coloration des fibres kératiniques qui comprend une étape de traitement du cuir chevelu à partir d'un ester de sorbitan polyoxyéthyléné particulier.

Il est connu de teindre les fibres kératiniques et en particulier les cheveux humains avec des compositions tinctoriales contenant des précurseurs de colorant d'oxydation, appelés généralement bases d'oxydation, tels que des ortho- ou para-phénylènediamines, des ortho- ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés. Les nuances obtenues avec ces bases d'oxydation peuvent être modifiées par l'addition de coupleurs ou modificateurs de coloration, ces derniers étant choisis notamment parmi les méta-diaminobenzènes aromatiques, les méta-aminophénols, les méta-diphénols et certains composés hétérocycliques tels que des composés indoliques et pyridiniques.

La coloration d'oxydation est généralement mise en oeuvre en présence d'agent alcalin qui favorise la coloration des fibres kératiniques.

La variété des molécules mises en jeu au niveau des bases d'oxydation et des coupleurs, permet l'obtention d'une riche palette de couleurs. La coloration dite "permanente" obtenue grâce à ces colorants d'oxydation, permet d'obtenir des nuances dans l'intensité souhaitée et présente une bonne tenue face aux agents extérieurs tels que la lumière, les intempéries, le lavage, les ondulations permanentes, la transpiration et les frottements.

Cependant, la coloration d'oxydation étant mise en oeuvre en présence d'un agent oxydant et d'agent alcalin, elle entraîne parfois une sensation d'inconfort se traduisant par des picotements et/ou des échauffements locaux du cuir chevelu.

Il est déjà connu de protéger les fibres kératiniques devant subir ou ayant subies une coloration à partir de précurseur de coloration notamment par l'utilisation de polymères particuliers. Cependant, cette protection n'est pas complètement satisfaisante, en particulier, elle peut entraîner une teinture moins puissante due à la présence de ces polymères.

Par ailleurs, il est déjà connu d'utiliser des esters de sorbitan polyoxyéthyléné dans des produits de coloration des fibres kératiniques. En particulier, le document DE 19923438 décrit l'utilisation d'esters de sorbitan polyoxyéthyléné pour réduire le tachâge du cuir chevelu au cours de la teinture.

Le document JP 03157320 décrit une composition de coloration des cheveux comprenant un pigment, une résine, un solvant organique et un ester de sorbitan polyoxyéthyléné du même type que celui de la présente invention.

Le but de la présente invention est de fournir un nouveau procédé de teinture des fibres kératiniques, en particuliers les fibres kératiniques humaines telles que les cheveux, qui permet de limiter l'inconfort lié à la coloration.

Ce but est atteint par la présente invention qui a pour objet un procédé de coloration des fibres kératiniques tels que les cheveux qui comprend dans l'ordre, l'application sur le cuir chevelu d'une composition comprenant un ester de sorbitan polyoxyéthyléné dont le nombre de moles d'oxyde d'éthylène est inférieur ou égal à 10, puis l'application sur les fibres kératiniques d'une composition de coloration comprenant dans un milieu approprié au moins un précurseur de colorant, l'étape d'application de la composition de coloration se faisant immédiatement après l'application de l'ester de sorbitan ou les deux applications étant décalées dans le temps, le décalage pouvant s'étendre jusqu'à 30 minutes.

Un tel procédé permet notamment de conserver une coloration puissante tout en limitant l'inconfort pouvant être ressenti au niveau du cuir chevelu au moment de l'application de la composition de coloration ou après cette application.

A titre d'exemple d'ester de sorbitan polyoxyéthyléné dont le nombre de moles d'oxyde d'éthylène est inférieur ou égal à 10, on peut citer le mono-laurate de sorbitan oxyéthyléné à 4OE ou polysorbate 21, le mono-stéarate de sorbitan oxyéthyléné à 4OE ou polysorbate 61, le mono-oléate de sorbitan oxyéthyléné à 5OE ou polysorbate 81. Ces esters de sorbitan sont par exemple commercialisés par la société Uniquema sous la dénomination Tween 21, Tween 61 ou Tween 81.

Selon un mode de réalisation particulier, le nombre de moles d'oxyde d'éthylène est de préférence inférieur à 6 moles d'oxyde d'éthylène. De préférence, le nombre de moles d'oxyde d'éthylène varie de 2 à 5 moles d'oxyde d'éthylène, bornes comprises.

Selon la présente invention, l'ester de sorbitan peut être présent dans la composition utile dans le procédé de l'invention en quantité très variable en fonction par exemple du type de coloration ou de la nature des fibres kératiniques à teindre. Selon un mode de réalisation particulier, la composition peut contenir une quantité d'ester de sorbitan variant de 0,01 à 20 % en poids par rapport au poids de la composition, plus particulièrement une quantité variant de 0,1 à 10% et de préférence une quantité variant de 1 à 8 %.

La composition contenant l'ester de sorbitan utile dans la présente invention peut contenir d'autres additifs classiquement utilisés en cosmétique et en particulier dans le domaine de la coloration capillaire. En particulier, cette composition peut contenir des agents anti-oxydants, des agents de pénétration, des agents séquestrants, des parfums, des tampons, des agents dispersants, des tensioactifs, des agents conditionneurs, des agents filmogènes, des épaississants, les céramides, des agents conservateurs, les agents nacrants ou opacifiants, les vitamines ou provitamines.

La composition contenant l'ester de sorbitan peut se présenter sous diverses formes telles que lotions, gels, crèmes, shampooings, sticks, mousses, sprays. Pour certaines de ces formes, elle peut être conditionnée en flacon pompe ou dans un récipient aérosol. Dans le cas de l'aérosol, la composition est associée à un agent propulseur qui peut être par exemple un alcane ou un mélange d'alcanes, du diméthyl éther, de l'azote, du protoxyde d'azote, du gaz carbonique ou des halogénoalcanes, ainsi que leurs mélanges.

Les précurseurs de colorants utiles dans la composition de coloration sont par exemple les bases d'oxydations et les coupleurs classiquement utilisés pour la coloration par oxydation.

A titre d'exemple, les bases d'oxydation peuvent être choisies parmi les para-phénylènediamines, les bis-phénylalkylènediamines, les para-aminophénols, les bis-para-aminophénols, les ortho-aminophénols, les bases hétérocycliques et leurs sels d'addition.

Parmi les para-phénylènediamines, on peut citer la para-phénylènediamine, la para-toluènediamine, la 2 chloro para-phénylènediamine, la 2,3-diméthyl para-phénylènediamine, la 2,6 diméthyl para-phénylènediamine, la 2,6-diéthyl para-phénylènediamine, la 2,5 diméthyl para-phénylènediamine, la N,N-diméthyl para-phénylènediamine, la N,N-diéthyl para-phénylènediamine, la N,N-dipropyl para-phénylènediamine, la 4 amino N,N diéthyl 3-méthyl aniline, la N,N-bis-(β-hydroxyéthyl) para-phénylènediamine, la 4-N,N-bis-(β-hydroxyéthyl)amino 2-méthyl aniline, la 4 N,N bis (β hydroxyéthyl)amino 2-chloro aniline, la 2 β hydroxyéthyl para-phénylènediamine, la 2 fluoro para-phénylènediamine, la 2 isopropyl para-phénylènediamine, la N (β hydroxypropyl) para-phénylènediamine, la 2 hydroxyméthyl para-phénylènediamine, la N,N diméthyl 3-méthyl para-phénylènediamine, la N,N (éthyl, β-hydroxyéthyl) para-phénylènediamine, la N-(β,γ-dihydroxypropyl) para-phénylènediamine, la N (4' aminophényl) para-phénylènediamine, la N phényl para-phénylènediamine, la 2 β hydroxyéthyloxy para-phénylènediamine, la 2-β-acétylaminoéthyloxy para-phénylènediamine, la N (β méthoxyéthyl) para-phénylène-diamine, la 4-aminophénylpyrrolidine, la 2-thiényl para-phénylènediamine, le 2-β hydroxyéthylamino 5-amino toluène, la 3-hydroxy 1-(4'-aminophényl)pyrrolidine et leurs sels d'addition avec un acide.

Parmi les para-phénylènediamines citées ci-dessus, la para-phénylènediamine, la para-toluènediamine, la 2 isopropyl para-phénylènediamine, la 2-β-hydroxyéthyl para-phénylènediamine, la 2 β hydroxyéthyloxy para-phénylène-diamine, la 2,6-diméthyl para-phénylènediamine, la 2,6-diéthyl para-phénylènediamine, la 2,3 diméthyl para-phénylènediamine, la N,N bis-(β-hydroxyéthyl) para-phénylènediamine, la 2 chloro para-phénylènediamine, la 2 β acétylaminoéthyloxy para-phénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3 diamino propanol, la N,N'-bis-(β-hydroxyéthyl) N,N'-bis-(4'-aminophényl) éthylènediamine, la N,N' bis-(4-aminophényl) tétraméthylènediamine, la N,N' bis (β hydroxyéthyl) N,N'-bis-(4-aminophényl) tétraméthylènediamine, la N,N' bis-(4-méthyl-aminophényl) tétraméthylènediamine, la N,N'-bis-(éthyl) N,N' bis (4'-amino, 3' méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4 amino 3 fluoro phénol, le 4 amino 3-hydroxyméthyl phénol, le 4-amino 2-méthyl phénol, le 4-amino 2 hydroxyméthyl phénol, le 4-amino 2-méthoxyméthyl phénol, le 4 amino 2 aminométhyl phénol, le 4-amino 2-(β-hydroxyéthyl aminométhyl) phénol, le 4 amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6 méthyl phénol, le 5-acétamido 2 amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5 diamino pyridine, la 2-(4-méthoxyphényl)amino 3 amino pyridine, la 2,3 diamino 6-méthoxy pyridine, la 2-(β-méthoxyéthyl)amino 3-amino 6-méthoxy pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5 a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs d'addition avec un acide ou avec une base.

Parmi les dérivés pyrimidiniques, on peut citer les composés décrits par exemple dans les brevets DE 2359399 ; JP 88 169571 ; JP 05-63124 ; EP 0770375 ou demande de brevet WO 96/15765 comme la 2,4,5,6-tétra-aminopyrimidine, la 4 hydroxy 2,5,6-triaminopyrimidine, la 2 hydroxy 4,5,6-triaminopyrimidine, la 2,4 dihydroxy 5,6-diaminopyrimidine, la 2,5,6 triaminopyrimidine, et les dérivés pyrazolo-pyrimidiniques tels ceux mentionnés dans la demande de brevet FR A 2750048 et parmi lesquels on peut citer la pyrazolo-[1,5-a]-pyrimidine-3,7-diamine ; la 2,5-diméthyl pyrazolo [1,5 a]-pyrimidine-3,7-diamine ; la pyrazolo-[1,5-a]-pyrimidine-3,5-diamine ; la 2,7 diméthyl pyrazolo-[1,5-a]-pyrimidine-3,5-diamine ; le 3-amino pyrazolo-[1,5 a]-pyrimidin-7-ol ; le 3-amino pyrazolo-[1,5-a]-pyrimidin-5-ol ; le 2 (3 amino pyrazolo-[1,5-a]-pyrimidin-7-ylamino)-éthanol, le 2 (7-amino pyrazolo-[1,5-a]-pyrimidin-3-ylamino)-éthanol, le 2-[(3-amino-pyrazolo[1,5-a]pyrimidin-7-yl)-(2 hydroxy-éthyl)-amino]-éthanol, le 2-[(7-amino-pyrazolo[1,5-a]pyrimidin-3-yl)-(2 hydroxy-éthyl)-amino]-éthanol, la 5,6-diméthyl pyrazolo-[1,5-a]-pyrimidine-3,7 diamine, la 2,6 diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5 diamino 1-méthyl pyrazole, le 4,5-diamino 1-(β-hydroxyéthyl) pyrazole, le 3,4 diamino pyrazole, le 4,5-diamino 1 (4' chlorobenzyl) pyrazole, le 4,5-diamino 1,3-diméthyl pyrazole, le 4,5 diamino 3-méthyl 1-phényl pyrazole, le 4,5-diamino 1-méthyl 3-phényl pyrazole, le 4-amino 1,3-diméthyl 5-hydrazino pyrazole, le 1 benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1 (β hydroxyéthyl) 3-méthyl pyrazole, le 4,5 diamino 1 éthyl 3-méthyl pyrazole, le 4,5-diamino 1-éthyl 3 (4' méthoxyphényl) pyrazole, le 4,5-diamino 1-éthyl 3 hydroxyméthyl pyrazole, le 4,5-diamino 3-hydroxyméthyl 1 méthyl pyrazole, le 4,5-diamino 3 hydroxyméthyl 1-isopropyl pyrazole, le 4,5 diamino 3-méthyl 1 isopropyl pyrazole, le 4-amino 5-(2'-aminoéthyl)amino 1,3 diméthyl pyrazole, le 3,4,5 triamino pyrazole, le 1-méthyl 3,4,5-triamino pyrazole, le 3,5-diamino 1 méthyl 4-méthylamino pyrazole, le 3,5-diamino 4 (β hydroxyéthyl)amino 1 méthyl pyrazole, et leurs sels d'addition avec un acide.

La ou les bases d'oxydation présentes dans la composition de coloration utile sont en général présentes chacune en quantité comprise entre 0,001 à 10 % en poids environ du poids total de la composition tinctoriale, de préférence entre 0,005 et 6 %.

Les coupleurs utiles dans la composition de coloration sont par exemple les coupleurs métaphénylènediamines, les coupleurs méta-aminophénols, les coupleurs métadiphénols, les coupleurs naphtaléniques, les coupleurs hétérocycliques et leurs sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans la composition de coloration utile dans la présente invention, le ou les coupleurs sont chacun généralement présents en quantité comprise entre 0,001 et 10 % en poids environ du poids total de la composition tinctoriale, de préférence entre 0,005 et 6 %.

La composition de coloration peut de plus comprendre des colorants directs. A titre de colorant direct, on peut citer les colorants directs nitrés benzéniques neutres, acides ou cationiques, les colorants directs azoïques neutres acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

Le milieu approprié pour la coloration est avantageusement un milieu cosmétique constitué par de l'eau ou par un mélange d'eau et d'au moins un solvant organique tel que, par exemple, les alcools inférieurs en C1-C4, ramifiés ou non, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, le glycérol ainsi que les alcools aromatiques comme l'alcool benzylique ou le phénoxyéthanol, et leurs mélanges.

Les solvants sont, de préférence, présents dans des proportions de préférence comprises entre 1 et 40 % en poids par rapport au poids total de la composition tinctoriale, et encore plus préférentiellement entre 5 et 30 % en poids.

La composition de coloration utile dans le procédé de l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes et en particulière des polymères fixants non ioniques, cationiques, anioniques, amphotères, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels adjuvants de manière telle que les propriétés avantageuses attachées intrinsèquement à la composition conforme à l'invention ne soient pas, ou substantiellement pas, altérées par la ou les adjonctions envisagées.

Le pH de la composition de coloration est généralement compris entre 2 et 12 environ, de préférence compris entre 6 et 12.

Il peut être ajusté à la valeur désirée au moyen d'agents acidifiants ou alcalinisants habituellement utilisés en coloration des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants, on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (III) suivante : 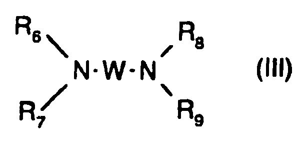 dans laquelle W est un reste propylène éventuellement substitué par un groupement hydroxyle ou un radical alkyle en C₁-C₄; R₆, R₇, R₈ et R₉, identiques ou différents, représentent un atome d'hydrogène, un radical alkyle en C₁-C₄ ou hydroxyalkyle en C₁-C₄.

La composition de coloration est généralement mise en oeuvre à partir d'une composition contenant un agent alcalin, cet agent alcalin étant en général présent en quantité supérieure à 5 % en poids par rapport au poids de la composition de coloration. Il peut être présent en des quantités supérieures à 10 %, en particulier supérieures à 15 %.

Selon un mode de réalisation particulier de l'invention, la composition de coloration utile dans le procédé de traitement des cheveux de l'invention comprend un agent alcalinisant, notamment de l'ammoniaque et/ou une alcanolamine tel que l'éthanolamine et/ou un silicate tel que le silicate de sodium.

La composition de coloration selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

La composition de coloration peut de plus comprendre un agent oxydant. Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

L'agent oxydant peut être ajouté à la composition de coloration juste au moment de l'emploi ou il peut être mis en oeuvre à partir d'une composition oxydante le contenant, cette composition étant appliquée simultanément ou séquentiellement à la composition de l'invention. La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12, et encore plus préférentiellement entre 6 et 12. Il peut être ajusté à la valeur désirée au moyen d'agents acidifiants ou alcalinisants habituellement utilisés en teinture des fibres kératiniques et tels que définis précédemment.

La composition de coloration qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

Selon la présente invention, le temps de pose de la composition contenant l'ester de sorbitan peut être compris entre quelques secondes par exemple 5 secondes et 60 minutes et de préférence entre 30 secondes et 45 minutes.

L'étape de traitement à partir de la composition comprenant l'ester de sorbitan peut être mise en oeuvre à une température pouvant varier de 10°C à 220 °C, de préférence de 10 °C à 70 °C, et de façon particulièrement préférée de 10 à 60 °C et plus particulièrement à la température ambiante.

L'étape de traitement des fibres à partir de la composition contenant l'ester de sorbitan peut être suivie d'une étape de rinçage avant application de la composition de coloration. Cependant, selon un mode de réalisation particulier, l'application de la composition contenant l'ester de sorbitan n'est pas suivie d'un rinçage avant l'application de la composition de coloration.

L'étape de coloration peut être mise en oeuvre de façon classique pendant un temps suffisant pour obtenir la coloration désirée. Le temps de pose est généralement compris entre 1 à 60 minutes environ, de préférence 5 à 60 minutes environ L'étape de coloration est suivie par une étape de rinçage.

L'étape de traitement à partir de l'ester de sorbitan et /ou l'étape de coloration du procédé de la présente invention peuvent être mis en oeuvre à température ambiante ou à des températures plus élevées par exemple en utilisant un sèche cheveux, un casque de séchage, un fer à lisser, etc.

L'étape d'application de la composition de coloration se fait soit immédiatement après l'application de l'ester de sorbitan, soit les deux applications sont décalées dans le temps, le décalage pouvant s'étendre jusqu'à 30 minutes. Selon un mode de réalisation préféré, ce décalage varie entre 30 secondes et 15 minutes.

Les exemples suivants sont destinés à illustrer l'invention. Celle-ci n'est cependant pas limitée à ces modes de réalisation.

### EXEMPLES

On a préparé la composition de traitement suivante (en grammes)

| | Ex 1 | Ex 2 |
|---|---|---|
| CHLORHYDRATE DE CHLORHEXIDINE | 0,02 | |
| P-HYDROXYBENZOATE DE METHYLE | 0,2 | |
| EAU DESIONISEE MICROBIOLOGIQUEMENT PROPRE | 88,28 | |
| ALCOOL CETYLSTEARYLIQUE (C16/C18 30/70) | 6 | |
| ALCOOL CETYLSTEARYLIQUE OXYETHYLENE (33 OE) | 1,5 | |
| MONO-LAURATE DE SORBITANE OXYETHYLENE (4 OE) | 4 | 100 |

Chacune de ces compositions est appliquée sur le cuir chevelu en quantité suffisante pour protéger le cuir chevelu. Cette application est suivie de l'application d'une composition de coloration telle que décrite ci-dessous (en grammes).

| | Exemple 1 | Exemple 2 |
|---|---|---|
| 1-METHYL-2,5-DIAMINO-BENZENE | 1.7 g | 0.5 g |
| 1-HYDROXY-4-AMINO-BENZENE | | 0.4 g |
| 1,3-DIHYDROXYBENZENE | 1 g | 0.25 g |
| 1-HYDROXY-3-AMINO-BENZENE | 0.07 g | |
| 1-BETA-HYDROXYETHYLOXY-2,4-DIAMINO-BENZENE DICHLORHYDRATE | 0.03 g | |
| 2-METHYL-1,3-DIHYDROXYBENZENE | 0.5 g | 0.3 g |
| 1-METHYL-2-HYDROXY-4-AMINO-BENZENE | | 0.25 g |
| 1-METHYL-2-HYDROXY-4-BETA-HYDROXYETHYLAMINO-BENZENE | | 0.05 g |
| 6-HYDROXYINDOLE | | 0.01 g |
| MONOETHANOLAMINE PURE | 5 g | |
| Ammoniaque à 20% de NH3 | | 15 g |
| Polyquaternium 6 commercialisé par Nalco | | 3 g |
| Polyquaternium 22 commercialisé par Nalco | 1.5 g | |
| Hexadimethrine Chloride, (Mexomere PO, Chimex) | 1.5 g | |
| Propyleneglycol | 10 | 10 |
| Carbopol 980 commercialisé par Noveon (acide polyacrylique réticulé) | 0.4 g | 0.4 g |
| Alcool laurique oxyethylene à 12 moles d'oxyethylene | 7.5g | 7.5g |
| Alcool oleocetylique oxyethylene à 30 moles d'OE | 6 g | 6 g |
| Alcool décylique oxyethylene à 3 moles d'OE | 8 g | 8 g |
| Acide laurique | 2.5g | 2.5g |
| Alcool cetylstearylique 50/50 | 10 g | 10 g |
| Silice pyrogénée hydrophobe | 1g | 1g |
| Monostearate de glycerol | 1 g | 1 g |
| Réducteur, antioxidant, sequestrant, parfum | qs | qs |
| Eau déminéralisée qs | 100g | 1 00g |
| | | |

| | Exemple 3 | Exemple 4 |
|---|---|---|
| 1-METHYL-2,5-DIAMINO-BENZENE | 1.7g | 0.007 g |
| 1-HYDROXY-4-AMINO-BENZENE | | 0.007 g |
| 1,3-DIHYDROXYBENZENE | 1g | 0.014 g |
| 1-HYDROXY-3-AMINO-BENZENE | 0.07 g | |
| 1-BETA-HYDROXYETHYLOXY-2,4-DIAMINO-BENZENE DICHLORHYDRATE | 0.03 g | |
| 2-METHYL-1,3-DIHYDROXYBENZENE | 0.5 g | |
| Ammoniaque à 20% de NH3 | 10 g | 20 g |
| Polyquaternium 6 commercialisé par Nalco, | | 3 g |
| Polyquaternium 22, comercialisé par Nalco | 1.5 g | |
| Hexadimethrine Chloride, Mexomere PO, Chimex | 1.5 g | |
| Acide oleique | 2.5 g | 2.5 g |
| Mélange d'alcool stéaryliques plus ou moins oxyethyléné (2 à 21 OE) | 15 | 15g |
| Alcool oléique | 1 g | 1 g |
| Monamid 972 commercialisé par Uniquema (Amide gras) | 3 | 5 |
| Glycérine | | 5 |
| POLYURETHANE-16 | 0.2g | 0.4 g |
| HYDROXYPROPYL METHYL CELLULOSE | 0.3 g | 0.7 g |
| Réducteur, antioxidant, sequestrant, parfum | qs | qs |
| Eau déminéralisée qs | 100g | 100g |

Les compositions de coloration 1 à 4 sont mélangées extemporanément avec de l'eau oxygénée dans les conditions décrites ci-dessous
Composition 1 : mélange avec 1 fois et demi leur volume d'eau oxygénée à 9 volumes
Compositions 2 et 3 : mélange avec 1 fois et demi leur volume d'eau oxygénée à 20 volumes
Composition 4 : mélange avec 2 fois leur volume d'eau oxygénée à 40 volumes.

Les mélanges ainsi réalisés sont appliqués sur des cheveux gris à 90% de blancs naturels à raison de 30 g pour 3 g de cheveux.

Après 30 minutes de pose, les cheveux sont rincés, lavés avec un shampooing standard puis rincées à nouveau.

La coloration capillaire est évaluée de manière visuelle.

| | **Hauteur de ton** | **Reflet** |
|---|---|---|
| composition 1 | Châtain | naturel |
| composition 2 | Blond foncé | Cuivré acajou |
| composition 3 | Châtain | naturel |
| composition 4 | Blond très très clair | naturel |

Les fibres ainsi obtenues présentent une coloration satisfaisante dans de bonnes conditions de confort pour le modèle.

## Revendications

1. Procédé de coloration des fibres kératiniques qui comprend dans l'ordre, une étape de traitement du cuir chevelu à partir d'une composition comprenant au moins un ester de sorbitan polyoxyéthyléné dont le nombre de moles d'oxyde d'éthylène est inférieur ou égal à 10, et une étape de coloration des fibres à partir d'une composition de coloration comprenant dans un milieu approprié au moins un précurseur de colorant, l'étape d'application de la composition de coloration se faisant immédiatement après l'application de l'ester de sorbitan ou les deux applications étant décalées dans le temps, le décalage pouvant s'étendre jusqu'à 30 minutes.

2. Procédé selon la revendication 1 dans lequel l'étape de traitement du cuir chevelu est mise en oeuvre à partir d'une composition comprenant de l'ester de sorbitan polyoxyéthyléné dont le nombre de moles d'oxyde d'éthylène est inférieur à 6 moles d'oxyde d'éthylène.

3. Procédé selon la revendication 1 ou 2 dans lequel l'étape de traitement du cuir chevelu est mise en oeuvre à partir d'une composition comprenant de l'ester de sorbitan polyoxyéthyléné dont le nombre de moles d'oxyde d'éthylène varie de 2 à 5 moles d'oxyde d'éthylène, bornes incluses.

4. Procédé selon la revendication 1, 2 ou 3 dans lequel l'ester de sorbitan polyoxyéthyléné est choisi parmi le mono-laurate de sorbitan oxyéthyléné à 4OE, le mono-stéarate de sorbitan oxyéthyléné à 4OE, le mono-oléate de sorbitan oxyéthyléné à 5OE.

5. Procédé selon l'une quelconque des revendications précédentes dans lequel la quantité d'ester de sorbitan varie de 0,01 à 20% en poids par rapport au poids de la composition, de préférence de 0,1 à 10 %.

6. Procédé selon l'une quelconque des revendications 1 à 5 dans lequel la quantité d'ester de sorbitan varie entre 1 et 8 %.

7. Procédé selon l'une quelconque des revendications précédentes dans lequel la composition contenant l'ester de sorbitan comprend des agents anti-oxydants, des agents de pénétration, des agents séquestrants, des parfums, des tampons, des agents dispersants, des tensioactifs, des agents conditionneurs, des agents filmogènes, des épaississants, des agents conservateurs, les agents nacrants ou opacifiants.

8. Procédé selon l'une quelconque des revendications précédentes dans lequel la composition de coloration comprend au moins une base d'oxydation choisie parmi les para-phénylènediamines, les bis-phénylalkylènediamines, les para-aminophénols, les bis-para-aminophénols, les ortho-aminophénols, les bases hétérocycliques et leurs sels d'addition.

9. Procédé selon la revendication 8 dans lequel la ou les bases d'oxydation présentes dans la composition de coloration sont en présentes chacune en quantité comprise entre 0,001 à 10 % en poids du poids total de la composition tinctoriale, de préférence entre 0,005 et 6 %

10. Procédé selon l'une quelconque des revendications précédentes dans lequel la composition de coloration comprend au moins un coupleur choisi parmi les coupleurs méta-phénylènediamines, les coupleurs méta-aminophénols, les coupleurs métadiphénols, les coupleurs naphtaléniques, les coupleurs hétérocycliques et leurs sels d'addition.

11. Procédé selon la revendication 10 dans lequel le ou les coupleurs sont chacun présents en quantité comprise entre 0,001 et 10% en poids du poids total de la composition tinctoriale, de préférence entre 0,005 et 6 %

12. Procédé selon l'une quelconque des revendications précédentes dans lequel la composition de coloration comprend de plus un colorant direct.

13. Procédé selon l'une quelconque de revendications précédentes dans lequel l'étape de traitement à partir de la composition contenant l'ester de sorbitan peut être mise en oeuvre pendant un temps de pose compris entre 5 secondes et 60 minutes.

14. Procédé selon l'une quelconque de revendications précédentes dans lequel l'étape de coloration peut être appliquée pendant un temps de pose compris entre 1 minutes et 60 minutes.

15. Procédé selon l'une quelconque de revendications précédentes dans lequel la composition de coloration contient un agent alcalinisant.

## Patentansprüche

1. Verfahren zum Färben von Keratinfasern, das folgende Schritte in der angegebenen Reihenfolge umfasst:
einen Schritt der Behandlung der Kopfhaut mit einer Zusammensetzung, die mindestens einen Ester von polyoxyethyleniertem Sorbitan enthält, bei dem die Molanzahl von Ethylenoxid kleiner als oder gleich 10 ist, und
einen Schritt der Färbung der Fasern mit einer Färbezusammensetzung, die in einem geeigneten Medium mindestens einen Farbstoffvorläufer enthält, wobei der Schritt der Aufbringung der Färbezusammensetzung sofort nach der Aufbringung des Sorbitanesters stattfindet oder die beiden Aufbringungen zeitlich versetzt sein können, wobei die Versetzung bis zu 30 Minuten betragen kann.

2. Verfahren nach Anspruch 1, bei dem der Schritt der Behandlung der Kopfhaut mit einer Zusammensetzung durchgeführt wird, die einen Ester von polyoxyethyleniertem Sorbitan enthält, bei dem die Molanzahl von Ethylenoxid kleiner als 6 Mol Ethylenoxid ist.

3. Verfahren nach Anspruch 1 oder 2, bei dem der Schritt der Behandlung der Kopfhaut mit einer Zusammensetzung durchgeführt wird, die einen Ester von polyoxyethyleniertem Sorbitan enthält, bei dem die Molanzahl von Ethylenoxid im Bereich von 2 bis 5 Mol Ethylenoxid liegt, wobei die Grenzen eingeschlossen sind.

4. Verfahren nach Anspruch 1, 2 oder 3, bei dem der Ester von polyoxyethyleniertem Sorbitan ausgewählt wird unter mit 4 EO oxyethyleniertem Sorbitanmonolaurat, mit 4 EO oxyethyleniertem Sorbitanmonostearat und mit 5 EO oxyethyleniertem Sorbitanmonooleat.

5. Verfahren nach einem der vorhergehenden Ansprüche, bei dem die Menge des Sorbitanesters im Bereich von 0,01 bis 20 Gew.-%, bezogen auf das Gewicht der Zusammensetzung, und vorzugsweise im Bereich von 0,1 bis 10 % liegt.

6. Verfahren nach einem der Ansprüche 1 bis 5, bei dem die Menge des Sorbitanesters im Bereich von 1 bis 8 % liegt.

7. Verfahren nach einem der vorhergehenden Ansprüche, bei dem die Zusammensetzung, die den Sorbitanester enthält, ferner Folgendes enthält: Antioxidationsmittel, Penetrationsmittel, Sequestrierungsmittel, Parfums, Puffer, Dispergiermittel, Tenside, Konditioniermittel, filmbildende Mittel, Verdickungsmittel, Konservierungsmittel, Perlglanzmittel oder Trübungsmittel.

8. Verfahren nach einem der vorhergehenden Ansprüche, bei dem die Färbezusammensetzung mindestens eine Oxidationsbase enthält, die ausgewählt ist unter p-Phenylendiaminen, Bis-phenylalkylendiaminen, p-Aminophenolen, Bis-p-aminophenolen, o-Aminophenolen und heterocyclischen Basen sowie den Additionssalzen dieser Verbindungen.

9. Verfahren nach Anspruch 8, bei dem die in der Färbezusammensetzung vorliegende(n) Oxidationsbase(n) jeweils in einer Menge von 0,001 bis 10 Gew.-%, bezogen auf das Gesamtgewicht der Färbezusammensetzung, und vorzugsweise in einer Menge von 0,005 bis 6 % vorliegen.

10. Verfahren nach einem der vorhergehenden Ansprüche, bei dem die Färbezusammensetzung mindestens einen Kuppler enthält, der unter folgenden Kupplern ausgewählt ist: m-Phenylendiaminen, m-Aminophenolen, m-Diphenolen, Naphthalinkupplern und heterocyclischen Kupplern sowie den Additionssalzen dieser Verbindungen.

11. Verfahren nach Anspruch 10, bei dem der oder die Kuppler jeweils in einer Menge im Bereich von 0,001 bis 10 Gew.-%, bezogen auf das Gesamtgewicht der Färbezusammensetzung, und vorzugsweise in einer Menge im Bereich von 0,005 bis 6 % vorliegen.

12. Verfahren nach einem der vorhergehenden Ansprüche, bei dem die Färbezusammensetzung ferner einen Direktfarbstoff enthält.

13. Verfahren nach einem der vorhergehenden Ansprüche, bei dem der Schritt der Behandlung mit der den Sorbitanester enthaltenden Zusammensetzung während einer Einwirkungsdauer von 5 Sekunden bis 60 Minuten durchgeführt werden kann.

14. Verfahren nach einem der vorhergehenden Ansprüche, bei dem der Schritt der Färbung während einer Einwirkungsdauer im Bereich von 1 Minute bis 60 Minuten angewandt werden kann.

15. Verfahren nach einem der vorhergehenden Ansprüche, bei dem die Färbezusammensetzung ein Mittel zum Alkalischmachen enthält.

## Claims

1. Process for dyeing keratin fibres, which comprises, in order, a step of treating the scalp using a composition comprising at least one polyoxyethylenated sorbitan ester with a number of moles of ethylene oxide of less than or equal to 10, and a step of dyeing the fibres using a dye composition comprising at least one dye precursor in a suitable medium, the step of applying the dye composition being carried out immediately after application of the sorbitan ester, or the two applications being offset in time, where the offset may extend up to 30 minutes.

2. Process according to Claim 1, in which the scalp treatment step is performed using a composition comprising polyoxyethylenated sorbitan ester with a number of moles of ethylene oxide of less than 6 mol of ethylene oxide.

3. Process according to Claim 1 or 2, in which the scalp treatment step is performed using a composition comprising polyoxyethylenated sorbitan ester with a number of moles of ethylene oxide ranging from 2 to 5 mol of ethylene oxide, limits inclusive.

4. Process according to Claim 1, 2 or 3, in which the polyoxyethylenated sorbitan ester is chosen from sorbitan monolaurate oxyethylenated with 4 EO, sorbitan monostearate oxyethylenated with 4 EO and sorbitan monooleate oxyethylenated with 5 EO.

5. Process according to any one of the preceding claims, in which the amount of sorbitan ester ranges from 0.01% to 20% and preferably from 0.1% to 10% by weight relative to the weight of the composition.

6. Process according to any one of Claims 1 to 5, in which the amount of sorbitan ester ranges between 1% and 8%.

7. Process according to any one of the preceding claims, in which the composition containing the sorbitan ester comprises antioxidants, penetrants, sequestrants, fragrances, buffers, dispersants, surfactants, conditioning agents, film-forming agents, thickeners, preserving agents, nacreous agents or opacifiers.

8. Process according to any one of the preceding claims, in which the dye composition comprises at least one oxidation base chosen from para-phenylenediamines, bis(phenyl)alkylenediamines, para-aminophenols, bis-para-aminophenols, ortho-aminophenols and heterocyclic bases, and the addition salts thereof.

9. Process according to Claim 8, in which the oxidation base(s) present in the dye composition is (are) each present in an amount of between 0.001% and 10% and preferably between 0.005% and 6% by weight relative to the total weight of the dye composition.

10. Process according to any one of the preceding claims, in which the dye composition comprises at least one coupler chosen from meta-phenylenediamine couplers, meta-aminophenol couplers, meta-diphenol couplers, naphthalene-based couplers and heterocyclic couplers, and the addition salts thereof.

11. Process according to Claim 10, in which the coupler(s) is (are) each present in an amount of between 0.001% and 10% and preferably between 0.005% and 6% by weight relative to the total weight of the dye composition.

12. Process according to any one of the preceding claims, in which the dye composition also comprises a direct dye.

13. Process according to any one of the preceding claims, in which the treatment step using the composition containing the sorbitan ester may be performed for a leave-on time of between 5 seconds and 60 minutes.

14. Process according to any one of the preceding claims, in which the dyeing step may be applied for a leave-on time of between 1 minute and 60 minutes.

15. Process according to any one of the preceding claims, in which the dye composition contains a basifying agent.
